# EUROPEAN PATENT APPLICATION

(11) **EP 1 832 877 A1**
(43) Date of publication of application: **12.09.2007**
(21) Application number: 05819904.3
(22) Date of filing: 26.12.2005
(51) Int. Cl.: G01N 33/68, G01N 21/78

(54) **METHOD OF EXAMINING ALZHEIMER'S DISEASE**

(30) Priority: 28.12.2004 JP 2004381800
(71) Applicant: National University Corporation Kanazawa University, Kanazawa-shi, Ishikawa 920-1164 (JP); University of Fukui, Fukui-shi, Fukui 910-8507 (JP)
(72) Inventor: YAMADA, Masahito, Kanazawa University, Kanazawa-shi, Ishikawa 9201192 (JP); ONO, Kenjiro, Kanazawa University, Kanazawa-shi, Ishikawa 9201192 (JP); NAIKI, Hironobu, University of Fukui, Yoshida-gun, Fukui 9101193 (JP)
(74) Representative: Feakins, Graham Allan
(86) International application number: PCT/JP2005/023781
(87) International publication number: WO 2006/070740

(57) **Abstract**

[Subject] It is to make it possible to simply and accurately examine Alzheimer's disease.

[Attaining Means] A reaction solution having an amyloid β-protein, a body fluid sampled from an examinee and a buffer solution mixed with one another is reacted and, after a polymerization reaction of the amyloid β-protein is brought to an equilibrium state, the degree of polymerization of the amyloid β-protein is examined. For example, the reaction solution after being reacted is mixed with a fluorochrome to detect the degree of coloration of the reaction solution, thereby examining the degree of polymerization of the amyloid β-proteins. The fluorochrome is thioflavin T or a derivative thereof. The body fluid is a cerebrospinal fluid, blood or a constituent of the blood.

## Description

### [Technical Field]

The present invention relates to a method for accurately and simply examining Alzheimer's disease.

### [Background Art]

Alzheimer' disease is the major cause of a progressive mental disorder from which elderly persons suffer in developed countries and is characterized by neuropathological lesions, such as neurofibrillary changes including emergence of abnormal structural proteins within a neuron or phosphrylated taus, deposition of senile plaques by production and deposition of amyloid β-protein peptide, disorganization and inflammatory reaction, and neuron disappearance. While various causes of progressing Alzheimer's disease are conceived, as one of them, deposition of amyloid β-proteins due to their polymerization and fibril formation is being raised. The amyloid β-proteins are generally produced in cells and can be detected from blood plasmas or cerebrospinal fluids (CSFs) of not only Alzheimer's disease patients, but also healthy subjects. In the case of non-Alzheimer's disease patients, the amount of the amyloid β-proteins to be deposited is nil or, if any, very small. Only in the case of Alzheimer's disease patients, an increase in production of amyloid β-proteins or a decrease in metabolism and excretion of amyloid β-proteins is induced at an early stage, then polymerization of amyloid β-proteins, deposition of a large quantity of amyloid plaques, etc. occur, and symptoms of dementia go on progressively.

Though the head interior image examination by a CT scan, an MRI, etc. has heretofore been widely made, there is no examination satisfying both the diagnostic precision and the specificity of Alzheimer's disease by itself. For this reason, it is under existing circumstances that the actual examination has adopted a combination of plural examinations. In addition, since the morphological image examination of the brain in the head is an indirect examination based on the morphological change in the brain, the dementia cannot necessarily be examined accurately. This examination is quite helpless relative to the early detection of Alzheimer's disease, particularly before emergence of the morphological change.

In view of these situations, attention has been focused on a technique of early detecting Alzheimer's disease with high accuracy. In recent years, besides the amyloid β-proteins, trace amounts of molecules pertinent to Alzheimer's disease, such as apolipoprotein E (apo E), apolipoprotein J (apo J), serum amyloid P component (SAP), transthyretin (TTR), α1-antichymotrypsin (ACT), α2-macrogrobulin (α2M), etc. have been found out in a CSF, and studies etc. on use of these trace amounts of molecules as examination markers have been made. However, the trace amounts of these molecules are, due to the small contents, insufficient in sensitivity and, furthermore, the causation between these molecules and the Alzheimer's disease has not necessarily been elucidated yet. These are problematic.

Under these circumstances, it has been desired that a technique of conducting accurate examination be developed. For example, the method for examining dementia by the immunity measurement sampling analyte, such as blood, from a patient and utilizing an antigen-antibody reaction between a polypeptide peculiar to a dementia patient and contained in the analyte and an antibody for the polypeptide to thereby measure the polypeptide has been proposed (refer, for example, to JP-A 2000-193661). According to this method, it is possible to examine the dementia including Alzheimer's disease with a trace amount of the analyte. In addition, the method to detect or monitor the agglutination of amyloid in a sample, which includes the stage of bringing agglutinative amyloid having fluorescent labels bound covalently thereto into contact with a sample and the stage of detecting the fluorescent labels bound to the sample as an index of the amyloid agglutination has been proposed, and it is suggested that this method is performed for the diagnosis of Alzheimer's disease, for example (refer, for example, WO-A 2001-515044).

Incidentally, it has been reported that the presence of a CSF can inhibits β-amyloid fibril formation in vitro (refer, for example to Wisniewski T, Castano E, Ghiso J, Frangione B. Crerobrospinal fluid inhibits Alzheimer beta-amyloid fibril formation in vitro. Ann Neurol 1993; 34: 631-3). In this report, artificially synthesized amyloid β-protein and a phosphate buffer solution or CSF (from an Alzheimer's disease patient or healthy subject) are mixed at a ratio of 1:1. The mixture is left at room temperature for up to 70 hours, and the fluorescence spectrometry assay system using thioflavin T is utilized to quantitatively determine the amyloid β-protein made fibrotic.

However, the examination method described in JP-A 2000-193661 cites amyloid β-protein as a polypeptide peculiar to a dementia patient (Alzheimer's disease patient). Since the amyloid β-protein exists also in a healthy subject, it has to be said that use thereof as an examination marker for Alzheimer's disease is insufficient from the standpoint of examination accuracy.

Also, in WO-A 2001-515044, amyloid β-protein subjected in advance to fluorescence labeling is agglutinated. However, a possibility of the bound fluorescent label having some effect on the agglutination characteristic of the amyloid β-protein cannot be denied, thus posing the same problem on examination accuracy. Furthermore, since the method described in WO-A 2001-515044 is premised on use of an optional brain tissue including cerebral cortex, cerebellum and hippocampal tissues, use of a blood vessel tissue or use of a tissue, such as neuron, a patient is hard-pressed to sample a sample. Therefore, it cannot be said that the prior art method is a simple examination method. Moreover, the prior art method requires a great amount of fluorescently labeled amyloid β-proteins to be synthesized before the examination, resulting in induction of various disadvantages, such as an increase in cost, examination cumbersomeness, etc.

Furthermore, in the report by Wisniewski T, *et al.,* polymerization of amyloid β-protein is performed in the presence of a CSF from Alzheimer's disease patient, a CSF from a healthy subject or a phosphate buffer solution. However, the report has merely reached the confirmation that the degree of β-amyloid fibril formation differs between the CSF and the phosphate buffer solution. In the report, there is no difference in result obtained between the CSF from Alzheimer's disease patient and that from a healthy subject, and there is neither description nor suggestion concerning the application of the CSFs and phosphate buffer solution to Alzheimer's disease examination. In the basic medical research field elucidating and studying the Alzheimer disease pathogenic mechanism, the experimental technique of autonomously polymerizing in the phosphate buffer solution the amyloid β-protein artificially synthesized as reported by Wisniewski T, *et al* has been utilized. However, it has not been conceived heretofore that the experimental technique is applied to the clinical medicine discipline actually examining Alzheimer's disease.

The present invention has been proposed in view of the conventional state of affairs, and the object thereof is to provide a method for examining Alzheimer's disease capable of simple and accurate examination of the Alzheimer's disease.

### [Disclosure of the Invention]

### [Means for solving the Problems]

In the brain of an Alzheimer's disease patient, the polymerization and deposition of amyloid β-protein proceed. There are various views on the failure to deposit amyloid β-protein in the brain of a non-Alzheimer's disease patient, which include an ascent in concentration or activity of a polymerization-promoting factor of amyloid β-protein, descent in concentration or activity of a polymerization inhibitory factor thereof and the presence of polymerization reactive nuclei in the brain of an Alzheimer's disease patient. In any event, the brain of an Alzheimer's disease patient has in place the kind of environment necessary to polymerize the amyloid β-protein unlike the brain of a non-Alzheimer's disease patient. Considering that a body fluid, such as CSF, blood, etc., reflects the environment of the brain tissue, the present inventors have continued their studies. As a consequence, they have succeeded in making it possible to make a difference in the degree of polymerization of amyloid β-protein between an Alzheimer's disease patient and a non-Alzheimer's disease patient through the procedure that comprises mixing the body fluid sampled from an examinee with appropriate doses (5 Vol.% or more, for example) of a phosphate buffer solution for the purpose of promoting fibril formation to prepare an examination solution, mixing the examination solution with an amyloid β-protein solution to obtain a mixture, heating the mixture to the same degree of temperature as the human body temperature, retaining the temperature to promote the polymerization reaction of the amyloid β-protein and sufficiently securing the reaction time until the reaction is brought to an equilibrium state. Based on the data of this difference, Alzheimer's disease patients and non-Alzheimer's disease patients were actually subjected to the examination of the present invention to obtain the clinical knowledge that it could be judged whether or not the patients suffered from Alzheimer's disease. The present invention has been perfected on the basis of this knowledge.

That is to say, the method of the present invention for examining Alzheimer's disease is characterized by reacting a reaction solution having an amyloid β-protein, a body fluid sampled from an examinee and a buffer solution mixed with one another and examining the degree of polymerization of the amyloid β-protein after the reaction of the polymerization of the amyloid β-protein is brought to an equilibrium state.

When the polymerization reaction of the amyloid β-protein progresses in the presence of a body fluid and appropriate doses of a buffer solution etc., the degree of polymerization (fibril formation) of the amyloid β-protein varies depending on the presence of polymerization-promoting factors, polymerization inhibitory factors and polymerization reactive nuclei of the amyloid β-protein contained in the body fluid of an examinee. When the reaction proceeds using the body fluid of an Alzheimer's disease patient, for example, β-amyloid fibrils eventually high in degree of polymerization (large in length) are formed or a great quantity of β-amyloid fibrils are eventually produced as compared with those of a non-Alzheimer's disease patient. This fact has been confirmed for the first time by the present inventors. Even when the degrees of polymerization of the amyloid β-protein are examined in the initial stage of reaction, for example, since no discernible difference therein of the body fluids between an Alzheimer's disease patient and a non-Alzheimer's disease patient is made, it is important to examine the degrees of polymerization of the amyloid β-proteins eventually obtained after the polymerization reaction is brought to an equilibrium state (length, number and amount of the β-amyloid fibrils produced). By so doing, it is possible to accurately judge whether or not the body fluid of an examinee falls in an environment ready to polymerize the amyloid β-proteins, i.e. whether or not the examinee suffers from Alzheimer's disease.

Furthermore, since a body fluid easy to sample is used as a sample in the present invention, this sampling is less burdensome for an examinee, such as an Alzheimer's disease patient, and enables the examination to be performed extremely simply. Though it is conceivable to examine the degree of polymerization of the amyloid β-proteins using a brain tissue collected from a patient, this collection conspicuously burdens the patient, lacks in simplification or convenience and is unpractical.

Incidentally, in order to distinguish Alzheimer's disease patients from non-Alzheimer's disease patients, as described above, it is extremely important to examine the degree of polymerization of amyloid β-proteins after the polymerization reaction is brought to an equilibrium state. It is meaningless to examine it at the initial or intermediate stage. In spite of this, the report by Wisniewski T *et al.* does not consider this point al all. In the report by Wisniewski T *et al.,* the amyloid β-proteins are polymerized in the presence of CSFs up to 70 hours. In this case, however, there is no difference in the degree of polymerization between Alzheimer's disease patients and non-Alzheimer's disease patients. It is thought that they have come to a conclusion that the CSFs contain amyloid β-protein-inhibitory factors and ceased the reaction test before the polymerization reaction is brought to an equilibrium state.

Also, in the present invention, a buffer solution is added in appropriate doses to a reaction solution with the aim of making a difference in the degree of polymerization of amyloid β-proteins between Alzheimer's disease patients and non-Alzheimer's disease patients. However, the report by Wisniewski T *et al.* merely touches upon mixing CSFs and amyloid β-proteins and has no description concerning the addition of a buffer solution.

Furthermore, in the Alzheimer's disease examination method according to the present invention, it is preferred that the reaction solution having already been subjected to reaction be mixed with a fluorochrome to detect the degree of coloration of the reaction solution, thereby examining the degree of polymerization of the amyloid β-proteins. By adding a fluorochrome to the reaction solution after the polymerization reaction of the amyloid β-proteins to detect the degree of light emission, it is made possible to accurately judge whether or not the function of the polymerization inhibitory factors (apo E or apo J, for example) of the amyloid β-proteins contained in the body fluid of an examinee becomes weakened, i.e. whether or not the examinee suffers from Alzheimer's disease.

Moreover, in the Alzheimer's disease examination method according to the present invention, it is preferred that the fluorochrome be thioflavin T or its derivatives. The thioflavin T and its derivatives have features of being linked specifically to β-amyloid fibrils that are polymers of amyloid β-proteins to induce coloration in accordance with the degree of polymerization of the amyloid β-proteins including the molecular weight and amount of production of the β-amyloid fibrils, for example. For this reason, use of thioflavin T and its derivatives as the fluorochrome enables the degree of β-amyloid fibril formation to be measured more accurately and simply and the accuracy of the Alzheimer's disease examination to be heightened.

### [Effects of the Invention]

The Alzheimer's disease examination method according to the present invention is extremely accurate as compared with the conventional examination method and has an advantage in that the sampling of the sample (analyte) is less burdensome for an examinee. According to the present invention, therefore, it is made possible to provide an accurate and simple Alzheimer's disease examination method very useful for early detection and early treatment of Alzheimer's disease. Furthermore, according to the Alzheimer's disease examination method of the present invention, it is made possible to grasp the degree of the dementia progress.

### [Brief Description of the Drawings]

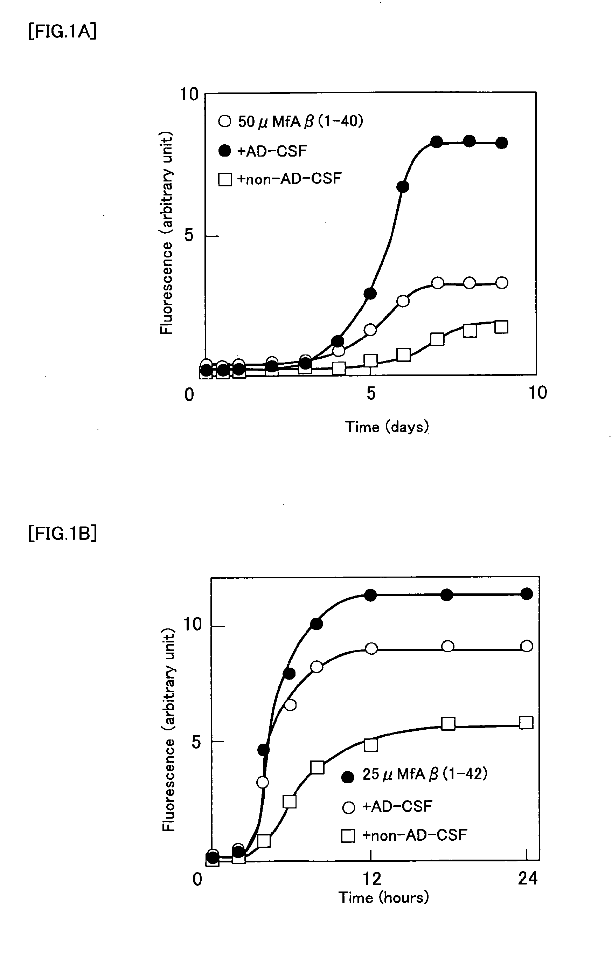

FIG. 1 shows characteristic diagrams illustrating the relationship between the progress of polymerization reaction of an amyloid β-protein and the fluorescence intensity by thioflavin T in a reaction solution, FIG. 1A being a characteristic diagram when using Aβ (1-40) as the amyloid β-protein and FIG. 1B being a characteristic diagram when using Aβ (1-42) as the amyloid β-protein.

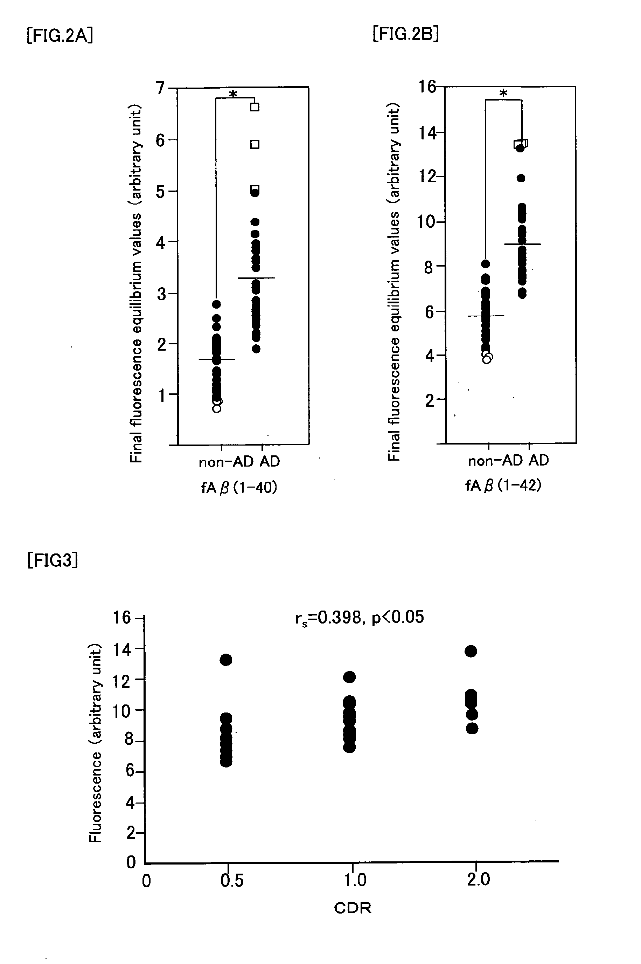

FIG. 2 shows diagrams having final fluorescence equilibrium values plotted thereon when the Aβ (1-40) and Aβ (1-42) have been polymerized in the presence of CSFs of an Alzheimer's disease patient (n = 40) and a non-Alzheimer's disease patient (n = 40), respectively, FIG. 2A being a diagram showing the level of formation of fAB (1-40) 9 days after the incubation thereof and FIG. 2B being a diagram showing the level of formation of fAB (1-42) 24 hours after the incubation thereof. Crossbars stand for mean values, respectively, and p < 0.001.
FIG. 3 is a characteristic diagram showing the correlation the final fluorescence equilibrium value of the formation of fAB (1-42) after the incubation using the CSF of an Alzheimer's disease patient and the clinical dementia rating (CDR).

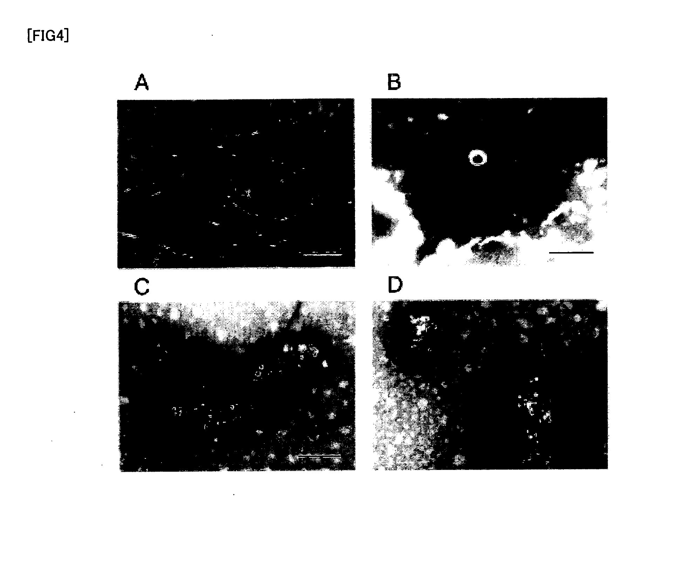

FIG. 4 shows electron micrographs illustrating the reaction solutions before or after a polymerization assay, A being a micrograph illustrating the reaction solution containing no CSF after the polymerization assay, B being a micrograph illustrating the reaction solution containing the CSF of an Alzheimer's disease patient after the polymerization assay, C being a micrograph illustrating the reaction solution containing the CSF of a non-Alzheimer's disease patient after the polymerization assay, and D being a micrograph illustrating the reaction solution containing the CSF of an Alzheimer's disease patient before the polymerization assay. The crossbar in each of these micrographs stands for a length of 250 nm.

### [Best Mode for carrying out the Invention]

The Alzheimer's disease examination method to which the present invention is applied will be described in detail herein below with reference to the accompanying drawings.

The Alzheimer's disease examination method to which the present invention is applied fundamentally comprises incubating a reaction solution having an amyloid β-protein, a body fluid sampled from an examinee and a buffer solution mixed with one another and, after a polymerization reaction of the amyloid β-protein is brought to an equilibrium state, examining the amyloid fibrils consequently produced, i.e. the degree of polymerization of the amyloid β-protein. The degree of polymerization of the amyloid β-protein is examined by mixing the reaction solution after being reacted and a fluorochrome and through examination of the degree of coloration of the reaction solution or through observation of the β-protein fibrils, which are reaction products, in the reaction solution with a microscope etc. For example, the correlation between the degree of coloration, such as color intensity, and the presence or the absence of Alzheimer's disease is obtained in advance, and it is possible to judge from the color intensity,, whether or not the Alzheimer's disease is present, based on the correlation.

First, in the present invention, an amyloid β-protein, a body fluid sampled from an examinee and a buffer solution are mixed with one another to prepare a reaction solution. To be specific, a body fluid sampled from an examinee and appropriate doses of a buffer solution are mixed with each other to prepare an examination solution, with which an amyloid β-protein solution is mixed, thereby preparing a reaction solution.

A human body fluid serving as a sample is at an advantage in small invasion when sampling it as compared with a brain tissue etc. While any of body fluids that can be sampled from an examinee, such as an Alzheimer's disease patient, is usable, blood or a cerebrospinal fluid (CSF) is preferably used because it is easy to sample. As the blood, besides the whole blood, a constituent of the blood, such as a blood plasma or blood serum, isolated from the whole blood, may be cited. It is preferred to use a CSF assumed to have an environment very close to that of a brain tissue and enabled to realize a more accurate examination. In the case of blood, it is preferred to use a constituent of the blood, such as a blood plasma or blood serum, isolated from the whole blood.

As the buffer solution, a neutral to weak alkaline buffer solution, such as a phosphate buffer solution, can be used. By having the buffer solution contained in the reaction solution, a polymerization reaction of the amyloid β-protein with the body fluid is prone to occurrence, with the result that a minimal change in the environment of body fluid, such as the degree of weakening the fibrosis-inhibiting factor contained in the body fluid, is amplified as the difference in degree of polymerization to enable more accurate examination to be performed. The examination solution obtained by mixing the buffer solution with the sampled body fluid is preferred to contain in the amount of 5 to 50 Vol. %. When the content of the buffer solution in the reaction solution falls short of 5 Vol. %, the shortage thereof possibly causes the polymerization reaction to be less prone to occurrence. In addition, the content of the buffer solution in the reaction solution is preferred to be 50 Vol. % or less. When the content of the buffer solution in the reaction solution exceeds the aforementioned range, there is a possibility of the amount of the amyloid fibrils produced being excessive. Incidentally, in the present invention, in order to make the variation in the amount of the β-amyloid fibrils produced in consequence of the polymerization reaction smaller, the reaction solution is heated to around the human body temperature and retained at that temperature. When it is intended to make the solution temperature lower than that temperature during the polymerization reaction, the content of the buffer solution may be more than 50 Vol. %.

As the amyloid β-protein, either a natural amyloid β-protein or a synthetic amyloid β-protein is usable. The synthetic amyloid β-protein is preferred to the natural amyloid β-protein. Amyloid β-proteins composed of 40 amino acids and of 42 amino acids have been known. Either one will do in the present invention. However, an amyloid β-protein composed of 42 amino acids is at an advantage in enabling the time required for the termination of the polymerization reaction to be shortened to around 1/10 that required when using an amyloid β-protein composed of 40 amino acids.

The amyloid β-protein is mixed with diluted ammonia water to an amyloid β-protein solution adjusted to pH 6 to 12, which is then mixed with the examination solution. The diluted ammonia water can readily dissolve the amyloid β-protein. Since the amyloid β-protein exists completely in the form of a monomer in an alkaline solution like a diluted ammonia water, by having the amyloid β-protein dissolved in the aqueous ammonia solution, a variation in results obtained during the course of the polymerization reaction can be made small.

The concentration of the amyloid β-protein in the reaction solution is preferred to be in the range of 5 µM to 100 µM. When the content of the amyloid β-protein falls short of the above range, the amount of β-amyloid fibrils produced will be too small. Inversely, when it exceeds the above range, the amount of β-amyloid fibrils produced will be too large to make it difficult to perform the quantitative measurement by fluorescence. It is noted, however, that even in this case, by lowering the temperature of the polymerization reaction, it is possible to use a solution having an amyloid β-protein concentration of more than 100 µM. However, use of such a solution may possibly prolong the time required for the polymerization reaction being brought to an equilibrium state or make a variation in the amount of β-amyloid fibrils to be produced larger.

The reaction solution having the amyloid β-protein, body fluid sampled from an examinee and buffer solution mixed with one another is then reacted to proceed with the polymerization reaction of the amyloid β-protein. The polymerization reaction of the amyloid β-protein comprises incubating the reaction solution, which is prepared by having the examination solution containing the body fluid and buffer solution mixed with the amyloid β-protein solution, for a prescribed time under conditions under which the amyloid β-protein is polymerizable.

It is preferred that the reaction solution be heated to a temperature in the range of 35°C to 40°C during the polymerization reaction. Though the polymerization reaction of the amyloid β-protein can be proceeded with even at room temperature, the incubation of the reaction solution under the conditions close to the in vivo environment, such as the temperature range as described above, enables a minimal change in the environment of body fluid, such as the degree of weakening the fibrosis-inhibiting factor contained in the body fluid, to be greatly amplified as the difference in degree of polymerization. As a result, it is possible to more accurately judge whether or not the disease under test is Alzheimer's disease. Moreover, the incubation of the reaction solution in the aforementioned temperature range enables a variation in the amount of produced β-amyloid fibrils to be made small.

It is also preferred that the reaction solution be adjusted during the polymerization reaction to pH 7.3 to pH 7.7. By adjusting the pH of the reaction solution in the above range, the reaction solution maintains its conditions close to the in vivo conditions to enable a minimal change in the environment of body fluid, such as the degree of weakening the fibrosis-inhibiting factor contained in the body fluid, to be greatly amplified as the difference in degree of polymerization. As a result, it is possible to more accurately judge whether or not the disease under test is Alzheimer's disease. By adjusting the pH of the reaction solution in the above range, it is also possible to make a variation in the amount of produced β-amyloid fibrils small.

Incidentally, the mechanism for producing β-amyloid fibrils is described using a polymer nucleation-dependent polymerization model comprised of a polymer nucleus formation reaction phase and an elongation phase of fibrils. According to this model, though the polymer nucleus formation reaction is thermodynamically hard to make and is at the rate-controlling step of the entire process, once a polymer constituting a polymerization nucleus is formed, the reaction is transferred to a fibril elongation reaction and subsequently first-order reaction model polymerization in which amyloid β-proteins are successively linked to the polymerization nuclei or to the stumps of the fibrils that have already existed in the reaction solution, with the conformation of the amyloid β-proteins varied proceeds instantly to form β-amyloid fibrils. The polymer nucleus formation reaction and fibril elongation reaction are readily made in vivo and also in a buffer solution in vitro.

Upon confirmation that the polymerization reaction of the amyloid β-protein has been brought to an equilibrium state, the incubation is terminated and the degree of polymerization of the amyloid β-protein in the reaction solution is examined. The degree of polymerization of the amyloid β-protein includes the length, molecular weight, number and amount of the β-amyloid fibrils produced. Preferably, the degree of polymerization of the amyloid β-protein is examined by a procedure comprising mixing the reaction solution that has been reacted with a fluorochrome, for example, and detecting the degree of coloration of the fluorochrome. In the present invention, the reaction solution not midway the polymerization reaction of the amyloid β-protein, but after the polymerization reaction is brought to an equilibrium state is mixed with a fluorochrome, to eliminate the affect by the rate of polymerization of the amyloid β-protein, thereby enabling accurate detection to be performed. Incidentally, the time required for the polymerization reaction of the amyloid β-protein being brought to an equilibrium state varies depending on the reaction conditions. It is determined in the present invention that the required time is 12 hours or more when using an amyloid β-protein composed of 42 amino acids and 7 days or more when using an amyloid β-protein composed of 40 amino acids.

When a fluorochrome for detecting β-amyloid fibrils is added to the reaction solution to measure color intensities at various reaction times and the measured color intensities are plotted, the plotted color intensities describe a sigmoid curve and finally reach equilibrium in consequence of the β-amyloid proteins in the reaction solution having been consumed, for example. The final color intensity shows the degree of polymerization of the β-amyloid fibrils finally produced by the polymerization reaction of the amyloid β-proteins, such as in the form of an increment or decrement of the factors for suppressing or promoting the polymerization of the amyloid β-proteins, varying depending on the environment of the body fluid. That is to say, the fact that the color intensity has reached the equilibrium means that the polymerization reaction of the amyloid β-proteins has been brought to an equilibrium state. Therefore, by mixing the reaction solution reacted for a period of time more than the time the color intensity has reached the equilibrium with the fluorochrome to link the fluorochrome to the β-amyloid fibrils and detecting the degree of coloration of the reaction solution, it is found that the degree of coloration of Alzheimer's disease patients is significantly higher than that of non-Alzheimer's disease patients. Thus, it is possible to determine based on this date whether or not the disease the patients have suffered from is Alzheimer's disease.

It is preferred to use thioflavin T as the fluorochrome for detecting β-amyloid fibrils. Derivatives of thioflavin T having an arbitrary group substituted for part of the thioflavin T are usable insofar as they have abilities to be linked and colored relative to the β-amyloid fibrils. Though detailed reasons therefore have not yet been necessarily made explicit, the thioflavin T and its derivatives have the specific features that they can be linked to β-amyloid fibrils precipitable onto neurons, i.e. amyloid β-proteins in a state in which the polymerization has progressed to a some extent, whereas they are not linked to amyloid β-proteins in a state of monomers or oligomers having a small degree of polymerization. Therefore, by measuring the fluorescence intensity of the thioflavin T or its derivatives added to the reaction solution having undergone the polymerization reaction, it is possible to accurately determine the level at which β-amyloid fibrils have been formed and furthermore the presence or absence of Alzheimer's disease. When using any other fluorochrome than the thioflavin T and its derivatives, there is a possibility of the examination accuracy being deteriorated.

The degree of polymerization of the reacted amyloid β-proteins can also be found through direct observation of the reaction solution in which amyloid β-proteins and the body fluid sampled from an examinee have been mixed with each other and which has undergone the reaction for a prescribed period of time, using an electron or fluorescent microscope, for example. Since Alzheimer's disease patients and non-Alzheimer's patients differ in number or mode of the β-amyloid fibrils finally produced, observation of this difference between the Alzheimer's patients and the non-Alzheimer's disease patients enables judgment on whether the patients suffer from Alzheimer's disease.

By polymerizing the amyloid β-proteins in the body fluid having the buffer solution mixed therewith and examining the degree of polymerization of the amyloid β-proteins after the polymerization reaction is brought to an equilibrium state, as described above, it is made possible to indirectly grasp the affect by polymerization-controlling factors including the polymerization-suppressing factor and polymerization-promoting factor of the amyloid β-proteins in the examinee (body fluid). Therefore, it is possible to accurately examine whether or not the body fluid has an environment in which the amyloid β-proteins are polymerizable, i.e. whether or not the examinee suffers from Alzheimer's disease.

In addition, since the examination method of the present invention uses a body fluid that can easily be sampled from an examinee as a sample, it is less burdensome for the examinee, very simple and useful for early diagnosis and early treatment of Alzheimer's disease. Furthermore, since the clinical test has revealed the correlation between the degree of polymerization of the amyloid β-proteins in the reaction solution having the buffer solution mixed therewith and the degree of progress of the symptom of dementia, the Alzheimer's disease examination method to which the present invention is applied makes it possible to grasp the degree of progress of the symptom of dementia.

### [Example]

A concrete example to which the present invention is applied will be described herein below based on experimental results. Incidentally, the present invention is not limited to the following example.

In the present example, experiments were conducted using cerebrospinal fluids (CSFs) obtained from both Alzheimer's disease patients and non-Alzheimer's disease patients. Furthermore, studies were made on the correlation between the clinical dementia rating (CDR) and the degree of formation of β-amyloid fibrils.

Twenty-two Japanese females and 18 Japanese males (age: 60 to 86, median age: 71.7) were examined as Alzheimer's disease patients. Incidentally, these patients satisfied the criteria of Diagnostic and Statistical Manual-IV and the criteria of NINCDS-ADRDA published by McKhann *et al.* (1984). Patients with the genetic-linkage were excluded. Patients with mild cognitive impairment (CDR = 0.5) were included when they later satisfied the criteria after progression.

Seventeen Japanese females and 23 Japanese males (age 60 to 83, median age: 70.1) were examined as non-Alzheimer's disease patients. Their diseases were diagnosed as cerebral infarction (one person), Parkinson's disease (one person), corticobasal degeneration (seven persons), progressive supranuclear palsy (three persons), diffuse lewy body dementia (two persons), Creutzfeld-Jakob disease (one person), multiple system atrophy (two persons), motor neuron disease including amyotrophic lateral sclerosis (six persons), multiple sclerosis (one person), myasthenia gravis (one person), meningitis (one person), muscle pain (three persons), epilepsy (one person), hepatic encephalopathy (one person), syndrome of inappropriate secretion of ADH (one person), malignant lymphoma (one person) and peripheral neuropathy (six persons).

Upon receipt of a written informed consent from each of the patients or their families, CSFs were sampled from both the Alzheimer's patients and the non-Alzheimer's patients. The CSFs were sampled by an ordinary lumbar puncture, then subjected to centrifugal separation at 1500 rpm for 10 minutes to be aliquoted and kept from deterioration at a temperature of minus 80degrees Celsius until their analysis.

The level of an amyloid β-protein contained in a CSF and composed of 42 amino acids (hereinafter referred to as "Aβ (1-42)") was measured by the sandwich enzyme-linked immunosorbent assay (ELISA). 21F12 that is a monoclonal antibody (Mab) specific for the C-terminus of Aβ (1-42) was used as a capturing agent, and 3D6 that is the N-terminal antibody of biotinylated monoclonal anti-Aβ (1-42) was used for the detection (INNOTEST β (1-42); Innogenetics, Gent, Belgium) (Vanderstichele *et al.* (1998); Andreasen *et al.* (1999)). There was no cross-reactivity with Aβ (1-40). Both the CSF samples and the standards were assayed in duplicate.

The concentration of the total tau in a CSF was determined by means of a highly sensitive sandwich ELISA using the Mab. AT120 was used as a capturing antibody, and two Mabs (HT7 and BT2), recognizing different epitopes were used as detection antibodies (INNOTEST hTAU-ag; Innogenetics, Gent, Belgium) (Vandermeeren *et al.* (1993); Blennow *et al.* (1995)). Both the CSF samples and the standards were assayed in duplicate.

Aβ(1-40) (lot number: 530108, Peptide Institute Inc.) and Aβ (1-42) (lot number: 521205, Peptide Institute Inc.) were used as the amyloid β-proteins, dissolved in an aqueous 0.02% ammonia solution in a room kept at 4 degrees Celsius, adjusted respectively to a concentration of 500 µM (2.2 mg/mt) and a concentration of 250 µM, and kept from deterioration at a temperature of -80°C. Fresh Aβ (1-40) and Aβ (1-42) obtained here were unfreezed, if necessary, and subjected to the following experiment.

The polymerization assay was performed in accordance with the report already published (Naiki *et al.* (1998)). First, was prepared a reaction mixture containing 50 µM of Aβ (1-40) or 25 µM of Aβ (1-42), 50 mM of a phosphate buffer solution (pH: 7.5), 100 mM of NaCl and 0 or 78 Vol. % of a CSF. 30 mℓ of the reaction mixture was introduced into each of oil-free PCR tubes (size: 0.5 mℓ, code number : 9046, Takara Shozo Co., Ltd., Otsu, Japan). The reaction tubes were placed in a DNA thermal cycler (PJ480, Perkin Elmer Cetus, Emeryville, California), and the temperature was elevated at the maximum speed from 4°C to 37°C. The heated reaction mixture was incubated for 0 to 9 days and, after an elapse of a prescribed length of time, the reaction tubes were cooled with ice to cease the reaction. During the incubation, the reaction tubes were left at rest. 5 µℓ of the reaction mixture was batched off from each reaction tube and subjected to measurement using a fluorescence spectrometer. This measurement was made three times per batched-off reaction mixture and the mean value was obtained.

The measurement of the fluorescence intensity was made, as described by Naiki and Nakakuki (1996), using a fluorescence spectrophotometer (Hitachi F-2500). The fluorescence intensities of the β-amyloid fibrils having the Aβ (1-40) polymerized (hereinafter referred to as fAB (1-40)) and β-amyloid fibrils having the Aβ (1-42) polymerized (hereinafter referred to as fAB (1-42)) were measured using an excitation wavelength of 445 nm and a fluorescence wavelength of 490 nm. A measurement sample solution contained 5 µM of thioflavin T (Wako Pure Chemical Industries, Ltd., Osaka, Japan) and 50 mM of a glycine-sodium hydroxide buffer solution (pH: 8.5).

The ROC (receiver operation characteristic) curve was analyzed, the area under the curve (AUC) was calculated, and the final fluorescence equilibrium values of the fAB (1-40) and fAB (1-42) after being incubated and the levels of the Aβ (1-40) and taus in the CSF were determined using the resultant values of the Alzheimer's disease patients and non-Alzheimer's disease patients. Incidentally the final fluorescence equilibrium values of the fAβ (1-40) and fAB (1-42) show the degrees of polymerization of the fAβs formed, respectively. For the analysis of the ROC curve and calculation of the AUC, Systatversion 10.0 (SPSS, Chicago, IL) was used to obtain the AUC, standard error (SE), sensitivity, characteristics and correct diagnosis ratio in accordance with the procedure of Hanley and McNail.

The comparison in fluorescence level of thioflavin T between the Alzheimer's disease patients and the non-Alzheimer's disease patients was made based on the unpaired *t* test with the Welch's correction. The Pearson's correlation coefficient and Spearman's correlation coefficient were calculated to perform correlation analyses. The correlation assumed when a p value was less than 0.05 was regarded as being significant.

The experimental results will be described hereinafter.
FIG. 1A is a correlation diagram between the time the Aβ (1-40) is incubated and the fluorescence intensity by thioflavin T in the reaction solution, and FIG. 1B is a correlation diagram between the time the Aβ (1-42) is polymerized and the fluorescence intensity in the reaction solution. In FIG. 1, the solid circle (•) shows the case where no CSF (0%) is added (n = 10), the blank circle (o) the case where the reaction mixture contains 78 Vol. % of CSF of an Alzheimer's disease patient (n = 40) and the blank square (□) the case where the reaction mixture contains 78 Vol. % of CSF of a non-Alzheimer's disease patient (n = 40).

As was clear from FIG. 1A, both the CSF sampled from the Alzheimer's disease patient (AD-CSF) and the CSF sampled from the non-Alzheimer's disease patient (non-AD-CSF) caused the final fluorescence equilibrium value of the formed fAβ (1-40) to be reduced. Also, as shown in FIG. 1B, the effect by the CSF of polymerization suppression on the fAβ (1-42) could be observed. That is to say, the non-Alzheimer's disease patient's CSF (non-AD-CSF) showed a higher effect of suppressing the formation of fAB (1-40) and fAB (1-42) than the Alzheimer's disease patient's CSF (AD-CSF).

Incidentally, as shown in FIG. 1, the fluorescence intensities by the thioflavin T after the incubation of fAβ (1-40) or fAB (1-42) described unique sigmoid curves. These curves were in accord with those of the polymer nucleation-dependent polymerization model (Jarrett and lansbury (1993); Naiki *et al.* (1997)). When the fAβ (1-40) and fAB (1-42) were stained with a red dye of Congo red, the observation thereof with a polarization microscope indicated typical birefringence of orangish-green color.

Diagrams having values of fluorescence by the thioflavin T, 9 days after the incubation, (of fAB (1-40)) and, 24 hours after the incubation, (of fAB (1-42)) plotted thereon, respectively, with respect to non-Alzheimer's disease patients and Alzheimer's disease patients are shown in FIG. 2.

As shown in FIG. 2A, the final equilibrium value of the fluorescence by the thioflavin in the formation of fAB (1-40) in the Alzheimer's disease patients' CSFs was 3.25 ± 1.04 (mean value ± standard deviation) and that in the non-Alzheimer's disease patients' CSFs was 1.63 ± 0.27, indicating that the value was significantly higher in the case of the Alzheimer's disease patients (p < 0.001). Also, as shown in FIG. 2B, that in the formation of fAB (1-42) in the Alzheimer's disease patients' CSFs was 9.00 ± 1.55 and that in the non-Alzheimer's disease patients' CSFs was 5.69 ± 1.02. Thus, similarly to the case of the formation of fAβ (1-40), the value was significantly higher in the case of the Alzheimer's disease patients (p < 0.001).

The value of the AUC obtained by the analysis of the ROC in the case where the final fluorescence equilibrium value of the fAβ (1-40) in the Alzheimer's disease patients' CSFs was 0.966 (SE = 0.018) and that of the fAB (1-42) was 0.980 (SE = 0.017), was higher than the value of the AUC obtained in the case of the Aβ (1-42) (0.867, SE = 0.042) and the tau (0.81, SE = 0.049). When the cutoff value of the final fluorescence equilibrium value in the formation of fAB (1-40) was set to be 2.11, the sensitivity, specificity and correct diagnosis ratio were 95%, 90% and 92.5%, respectively. Similarly, when the cutoff value of the final fluorescence equilibrium value in the formation of fAB (1-42) was set to be 7.36, the sensitivity, specificity and correct diagnosis ratio were 92.5%, 92.5% and 92.5%, respectively.

As described in the foregoing, by proceeding with the polymerization of amyloid β-proteins in the presence of CSFs and detecting with a fluorochrome, such as thioflavin T, β-amyloid fibrils consequently formed, it was made clearly possible to perform an accurate examination of Alzheimer's disease excellent in any of the accuracy, specificity and correct diagnosis ratio. That is to say, the examination method of the present invention proved to be superior to the method for measuring the levels of Aβ (1-42) and taus in CSFs that has recently been utilized as an auxiliary diagnostic method in terms of the examination and diagnosis of Alzheimer's disease.

FIG. 3 shows the correlation between the final fluorescence equilibrium value of the formed fAβ after the incubation using the CSFs of the Alzheimer's disease patients and the clinical dementia rating (CDR). As shown in FIG. 3, the final fluorescence equilibrium value of the formed fAB (1-42) after the incubation using the CSFs of the Alzheimer's disease patients and the CDR had significant correlation (rₛ = 0.398, p < 0.05). Thus, since the CDR that is one of evaluations for the degree of the general progress of dementia has a correlation with the final fluorescence equilibrium value of the formed fAβ after the incubation using the CSFs of the Alzheimer's disease patients, it has made it clear that the Alzheimer's disease examination method of the present invention can also measure the degree of progress of dementia.

The results of analysis of the reaction solution after the polymerization assay with an electron microscope will be described with reference to FIG. 4. FIG. 4A is a micrograph showing the reaction solution prepared to contain 50 µM of Aβ (1-40), 50 mM of phosphate buffer solution (pH: 7.5) and 100 mM of NaCl and incubated at 37°C for nine days. In the case where the reaction solution was incubated, with no CSF added thereto, fibrils were clearly observed as shown in FIG. 4A. It can be conceived that these fibrils have a nonbranched, helical filament structure having a helical periodicity of approximately 220 nm and an approximately 7 nm width.

Furthermore, a reaction solution prepared to contain 50 µM of Aβ (1-40), 50 mM of phosphate buffer solution (pH: 7.5), 100 mM of NaCl and a CSF of an Alzheimer's disease patient or non-Alzheimer's disease patient (78%) was incubated at 37°C for nine days. An electron microgram taken when using the CSF of the Alzheimer's disease patient is shown in FIG. 4B and that taken when using the CSF of the non-Alzheimer's disease patient is shown in FIG. 4C. In the case of the incubation with the Alzheimer's disease patient's CSF added, as shown in FIG. 4B, a great number of finely sheared fibrils were observed. A similar conformation could be observed also in the case where two other Alzheimer's disease patients' CSFs were used. On the other hand, in the case of the incubation with the non-Alzheimer's disease patient's CSF added, as shown in FIG. 4C, no discernible fibril could be observed while small and amorphous agglomerates were occasionally observed. A similar conformation could be observed also in the case where two other non-Alzheimer's disease patients' CSFs were used. When having made experiments using the Aβ (1-42), though the data thereof were not shown here, the same results as in the case of using the Aβ (1-40) were obtained. FIG. 4D is a microgram taken before the polymerization assay of the reaction solution containing the Alzheimer's disease patient's CSF. The same conformation as in FIG. 4D could be observed also in the case of using the non-Alzheimer's disease patient's CSF.

As described above, it was found that the fibrils different in conformation were eventually formed between the case where the polymerization of an amyloid β-protein was proceeded with using an Alzheimer's disease patient's CSF and the case where the polymerization of an amyloid β-protein was proceeded with using a non-Alzheimer's disease patient's CSF and that the results examined in degree with an electron microscope could be utilized for the examination of Alzheimer's disease.

## Claims

1. A method for examining Alzheimer's disease, comprising the steps of mixing an amyloid β-protein, a body fluid sampled from an examinee and a buffer solution to prepare a reaction solution, subjecting the amyloid β-protein to polymerization reaction in the reaction solution and examining a degree of polymerization of the amyloid β-protein after the polymerization reaction is brought to an equilibrium state.

2. A method for examining Alzheimer's disease according to claim 1, further comprising the step of mixing a fluorochrome to the reaction solution after the polymerization reaction is brought to the equilibrium state to detect a degree of coloration of the reaction solution, thereby examining the degree of polymerization of the amyloid β-protein.

3. A method for examining Alzheimer's disease according to claim 2, wherein the fluorochrome is thioflavin T or a derivative thereof.

4. A method for examining Alzheimer's disease according to any one of claims 1 to 3, wherein the body fluid is a cerebrospinal fluid, blood or a constituent of the blood.

5. A method for examining Alzheimer's disease according to any one of claims 1 to 4, wherein the reaction solution has a concentration of the amyloid β-protein in a range of 5 µM to 100 µM.

6. A method for examining Alzheimer's disease according to any one of claims 1 to 5, wherein the reaction solution is prepared by adding an amyloid β-protein solution to an examination solution having the body fluid and the buffer solution mixed with each other.

7. A method for examining Alzheimer's disease according to claim 6, wherein the examination solution contains the buffer solution in an amount of 5 to 50 Vol. %.

8. A method for examining Alzheimer's disease according to claim 6 or claim 7, wherein the amyloid β-protein solution is a solution having the amyloid β-protein dissolved in an aqueous ammonia solution.

9. A method for examining Alzheimer's disease according to any one of claims 1 to 8, wherein the polymerization reaction comprises heating the reaction solution to a temperature in a range of 35°C to 40°C.
